**Europäisches Patentamt**

**European Patent Office**

**Office européen des brevets**

(19)

(11) Veröffentlichungsnummer: **0 042 983**
**B1**

(12) # EUROPÄISCHE PATENTSCHRIFT

(45) Veröffentlichungstag der Patentschrift:
02.11.83

(51) Int. Cl.³: **C 07 C 19/045, C 07 C 17/02**

(21) Anmeldenummer: **81104206.8**

(22) Anmeldetag: **02.06.81**

(54) Verfahren zur Herstellung von 1,2-Dichloräthan.

(30) Priorität: **28.06.80 DE 3024610**

(43) Veröffentlichungstag der Anmeldung:
**06.01.82 Patentblatt 82/1**

(45) Bekanntmachung des Hinweises auf die Patenterteilung:
**02.11.83 Patentblatt 83/44**

(84) Benannte Vertragsstaaten:
**BE DE FR GB NL**

(56) Entgegenhaltungen:
**DE - A - 2 156 190**
**DE - A - 2 251 696**
**DE - A - 2 649 533**
**DE - B - 2 733 502**

(73) Patentinhaber: **BASF Aktiengesellschaft,
Carl-Bosch-Strasse 38, D-6700 Ludwigshafen (DE)**

(72) Erfinder: **Danz, Eckehard, Dr.,
Hermann-Hesse-Strasse 42, D-6700 Ludwigshafen (DE)**
Erfinder: **Birnbaum, Eberhard, Osloer Weg 26,
D-6700 Ludwigshafen (DE)**
Erfinder: **Krome, Gerd, Dr., Am Wingertsberg 28,
D-6719 Weisenheim (DE)**
Erfinder: **Stahnecker, Erhard, Dr., Oberer Rainweg 36,
D-6900 Heidelberg (DE)**

ACTORUM AG

Verfahren zur Herstellung von 1,2-Dichloräthan

Die Erfindung betrifft ein Verfahren zur Herstellung von 1,2-Dichloräthan durch Umsetzen von Äthylen mit Chlor in Gegenwart von Katalysatoren.

In der Technik haben sich vor allem zwei Verfahren zur Herstellung von 1,2-Dichloräthan eingeführt, die grosstechnische Anwendung finden.

Nach einem Verfahren wird Äthylen in flüssiger Phase in Gegenwart von Katalysatoren z.B. von Eisen-III-Chlorid mit Chlor umgesetzt. Nach einem anderen Verfahren erfolgt die Umsetzung von Äthylen mit Chlorwasserstoff und Sauerstoff in der Gasphase in Anwesenheit von Kupferchlorid enthaltenden Katalysatoren. Bei diesen Verfahren fallen Abgase an, die nicht umgesetztes Äthylen neben grössen Mengen Stickstoff, Kohlenmonoxid, Kohlendioxid, Chlorwasserstoff sowie kleineren Mengen von Ethylchlorid und Vinylchlorid enthalten.

Es bestand der Wunsch auch das Äthylen im Abgas mit Chlor zu Dichloräthan umzusetzen. Es haben sich deshalb grosstechnische Verfahren eingeführt, bei denen in der Gasphase das Äthylen enthaltende Abgas mit Chlor in technisch aufwendigen Apparaten umgesetzt wird. Bei diesen Verfahren, das beispielsweise in der DE-PS 17 93 051 (= GB-PS 1 230 604) beschrieben ist, wird das Äthylen weitgehend zu Dichloräthan umgesetzt. Es entstehen aber eine Reihe unerwünschter Nebenprodukte.

Aus der DE-OS 22 51 696 ist ein Verfahren bekannt, bei dem man die Ethylen enthaltenden Abgase der Oxichlorierung mit einem Flüssigkeitsgemisch aus Ethylendichlorid und Chlor umsetzt. Bei der dort beschriebenen Arbeitsweise ist es jedoch nachteilig, dass man Wasser aus dem Abgas der Oxichlorierung weitgehendst entfernen muss. Ausserdem bringt das Verfahren den Nachteil, dass relativ hohe Anteile an unerwünschten Nebenprodukten erhalten werden.

Aufgabe der Erfindung war es daher, ein Verfahren zu schaffen, bei dem die Umsetzung von Äthylen zu Dichloräthan zu einem grösseren Umsatz erfolgt und die Entstehung unerwünschter Nebenprodukte weitgehend vermieden wird.

Die Aufgabe wurde erfindungsgemäss gelöst durch ein Verfahren, bei dem man am oberen Ende einer Reaktionszone, deren Länge das 5 bis 30-fache des Querschnittsdurchmessers beträgt, worin ein 0,3 bis 30 Volumenprozent Äthylen enthaltendes Gasgemisch enthalten ist, ein feinteiliges Flüssigkeitsgemisch aus 1,2-Dichloräthan und Chlor einbringt, wobei auf 1000 Liter des Gasgemisches 5 bis 25 Liter des Flüssigkeitsgemisches entfallen und Chlor im Flüssigkeitsgemisch in solchen Mengen vorhanden ist, dass 1 bis 1,2 Mol Chlor auf 1 Mol Äthylen entfallen und die Temperatur in der Reaktionszone zwischen 0 und 90 °C gehalten wird, die durchschnittliche Verweilzeit des Gasgemisches zwischen 0,2 und 10,0 Sekunden beträgt und am Ende der Reaktionszone das Reaktionsgemisch abzieht und aufarbeitet.

Das Verfahren hat den Vorteil, dass die Umsetzung von Äthylen mit Chlor nahezu quantitativ verläuft, obwohl die Umsetzung von Äthylen mit Chlor bei der Flüssigphase bereits sehr hoch ist. In den Abgasen sind dennoch nur noch geringe Mengen an Äthylen, beispielsweise Mengen unter 1000 ppm und praktisch kein Chlor vorhanden. Es ist ein weiterer Vorteil des Verfahrens, dass man auch solche äthylenhaltigen Gase umsetzen kann, die ausser Äthylen beispielsweise Wasserdampf enthalten. Bei dem vorgenannten Verfahren nach DE-PS 17 93 051 muss Wasser von der Umsetzung entfernt werden. Darüber hinaus enthalten die Umsetzungsprodukte nach der erfindungsgemässen Verfahren nur geringe Mengen an unerwünschten Nebenprodukten. Auch werden lediglich einfache Apparate benötigt, die sich einfach warten lassen. Die in einem Apparat gegebenen Dimensionen umzusetzenden Gasmengen können in weiten Grenzen variiert werden.

Man kann nach dem Verfahren der Erfindung äthylenhaltige Gasströme einsetzen, die 0,3 bis 30 Volumenprozent Äthylen enthalten. Das Verfahren eignet sich insbesondere für die Chlorierung von Äthylen in solchen Abgasströmen mit 0,5 bis 10 Volumenprozent Äthylengehalt. Vorteilhaft eignen sich die Abgase von Direktchlorierungsverfahren von Äthylen bzw. von Oxichlorierungsverfahren, deren Äthylengehalt im obengenannten Bereich liegt. Ausser Äthylen können in den Gasströmen Kohlendioxid, Kohlenmonoxid, Stickstoff, Sauerstoff, chlorwasserstoffsaure oder chlorierte Kohlenwasserstoffe enthalten sein.

Man hat bei dem Verfahren dafür Sorge zu tragen, dass auf 1000 Liter Gas (normal Liter bei 0 °C) 5 bis 25 Liter, insbesondere 10 bis 20 Liter des Flüssigkeitsgemisches entfallen. Der Gasstrom wird vorteilhaft am oberen Ende der Reaktionszone eingebracht (Gleichstromwäsche). Es ist aber auch möglich, an anderen Stellen der Reaktionszone, z.B. am unteren Ende den Gasstrom in die Reaktionszone einzuschleusen (Gegenstromwäsche). Die Flüssigkeit wird in die Reaktionszone eingesprüht. Der Teilchendurchmesser der Flüssigkeitsteilchen soll kleiner als 500 µ betragen. Dies erreicht man z.B. durch Versprühen des Flüssigkeitsgemisches mittels eines Strahlwäschers. Die Teilchengrössenverteilung hängt vom Durchmesser der Düsen und vom Düsenvordruck ab.

Das Chlor und das Äthylendichlorid kann als Flüssigkeitsgemisch eingebracht werden. Es ist aber auch möglich, Chlor und Äthylendichlorid in einem Strahlwäscher zu mischen und gemeinsam zu versprühen. Die Menge Chlor im Flüssigkeitsgemisch ist so abgestimmt, dass ein Mol bis 1,2 Mole Chlor auf 1 Mol Äthylen entfallen. Man erreicht dadurch, dass bei der Chlorierung keine unerwünschten Nebenprodukte entstehen. Die Chlorierung des Restäthylens wird mit Hilfe üblicher Katalysatoren vorgenommen. Als solche eignen sich z.B. Metallchloride wie Antimonchlorid, Kupferchlorid und insbesondere Eisen-III-chlorid. Die Katalysatoren werden dem Flüssigkeitsgemisch beigegeben. Sie sind in dem Flüssigkeitsgemisch zwischen 10 und 1000 ppm enthalten. Es handelt sich dabei um die übliche bei der Chlorierung von Äthylen vorhandene Katalysatorkonzentration.

Die Temperatur des Flüssigkeitsgemisches wird zweckmässigerweise so gewählt, dass sich in der Reaktionszone eine Temperatur zwischen 0 und 90, insbesondere zwischen 10 und 50 °C einstellt. Es handelt sich dabei um eine mittlere Temperatur, die man durch Messen der Reaktionstemperatur an verschiedenen Stellen des Reaktionssystems ermittelt. Der Druck in der Reaktionszone ist nicht bestimmend für die Umsetzung der Reaktionspartner, zweckmässig arbeitet man bei 1 bar oder grösser. Aus technischen und Zweckmässigkeitsgründen ist es meist nicht angebracht, einen Druck über 10 bar zu wählen, jedoch kann auch bei höheren Drucken gearbeitet werden.

Die Reaktionszone ist so ausgestaltet, dass die Länge das 5- bis 30-fache des Durchmessers der Querschnittsfläche beträgt. Hat der Querschnitt eine andere als kreisförmige Fläche, so ist der Durchmesser einer entsprechend gleich grossen kreisförmigen Durchschnittsfläche zu verstehen.

Man regelt die Gasgeschwindigkeit so, dass eine Verweilzeit des Gases zwischen 0,2 und 10 Sekunden in der Reaktionszone erreicht wird. Vorteilhaft arbeitet man bei Verweilzeiten zwischen 0,5 und 5,0 Sekunden.

Die Reaktionsprodukte also fast ausschliesslich das 1,2-Dichloräthan werden aus dem unteren Ende der Reaktionszone ausgetragen und flüssigen von gasförmigen Bestandteilen durch Auskondensieren getrennt. Die demnach verbleibenden Abgase enthalten weniger als 1000 ppm Äthylen und praktisch kein Chlor, so dass sie direkt in den Abluftkanal geleitet werden können.

Nach einer besonders vorteilhaften Ausführungsform wird das aus der Reaktionszone austretende Reaktionsgemisch noch einmal mit 1,2-Dichloräthan gewaschen, wobei zweckmässig 2 bis 20 Liter, insbesondere 5 bis 10 Liter 500 m³ Abgas entfallen sollen. Mit dieser Massnahme erreicht man, dass eventuell vorhandene geringe Chlormengen aus dem Abgas ausgewaschen werden.

Beispiele

Wenn ein Rekationsgefäss mit kreisförmigem Querschnitt mit Durchmesser von 0,25 und einer Länge von 2,0 Metern hat, werden am oberen Ende äthylenhaltige Gasgemische eingebracht. Es werden ausserdem mittels einer Strahldüse Flüssigkeitsgemische aus 1,2-Dichloräthan und Chlor versprüht. Am unteren Ende des Reaktionsgefässes werden die gasförmigen und flüssigen Reaktionsprodukte bzw. Einsatzstoffe abgezogen und einer Waschkolonne zugeführt. In der Waschkolonne werden die Reaktionsprodukte mit 1,2-Dichloräthan gewaschen. Die flüssigen Produkte werden am Boden der Waschkolonne abgezogen und weiter aufgearbeitet.

In den folgenden Tabellen werden die jeweils eingesetzten Gasmengen, der Äthylengehalt, die Mengen an Chlor und an 1,2-Dichloräthan sowie die Druck- und Temperaturbedingungen angegeben. In einigen Fällen werden ausserdem die Angaben an Verunreinigungen, die im Reaktionsprodukt enthalten sind, aufgeführt.

Tabelle

| | | 1 | 2 | 3 | 4 |
|---|---|---|---|---|---|
| Gasmenge | m³/h | 220 | 500 | 500 | 100 |
| Reaktionstemp. | °C | 20 | 16 | 10 | 50 |
| Äthylengehalt des Gases | Vol.% | 3,2 | 3,5 | 3,5 | 23 |
| Chlor:Äthylen | Molverhältnis | 1:1 | 1,01:1 | 1:1 | 1:1 |
| Druck | bar | 1 | 5 | 1 | 1 |
| Katalysator | ppm | 500 | 500 | 400 | 700 |
| $C_2H_4$ nach Wäsche | Vol.% | 0,1 | 0,1 | 0,2 | 0,14 |
| 1,2-Dichloräthan | m³/h | 5 | 8 | 8 | 2 |
| Gas: Flüssigkeit | Liter/Liter | 0,022 | 0,016 | 0,016 | 0,04 |
| | | | | | |
| Analyse | Vol.% | | | | |
| Ethylchlorid | | | 0,05 | | |
| Tetrachlorkohlenstoff | | | 0,01 | | |
| Chloroform | | | 0,04 | | |
| 1,2-Dichloräthan | | | 99,1 | | |
| 1.1.2-Trichlorethan | | | 0,4 | | |
| Pentachlorethan | | | 0,4 | | |

## Patentansprüche

1. Verfahren zur Herstellung von 1,2-Dichlorethan durch Umsetzen von Ethylen mit Chlor in Gegenwart von Katalysatoren, bei dem man am oberen Ende einer Reaktionszone, deren Länge das 5- bis 30-fache des Querschnittsdurchmessers beträgt, worin ein 0,3 bis 30 Volumenprozent Ethylen enthaltendes Gasgemisch enthalten ist, ein Flüssigkeitsgemisch aus 1,2-Dichlorethan und Chlor einbringt, wobei auf 1000 Liter des Gasgemisches 5 bis 25 Liter des Flüssigkeitsgemisches entfallen, und Chlor im Flüssigkeitsgemisch in solchen Mengen vorhanden ist, dass 1 bis 1,2 Mol Chlor auf 1 Mol Ethylen entfallen und die Temperatur in der Reaktionszone zwischen 0 und 90 °C gehalten wird, dadurch gekennzeichnet, dass der Teilchendurchmesser der Teilchen des Flüssigkeitsgemisches kleiner als 500 µ und die durchschnittliche Verweilzeit des Gasgemisches zwischen 0,2 und 10,0 Sekunden beträgt.

2. Verfahren nach Anspruch 1, dadurch gekennzeichnet, dass man ein 0,5 bis 10 Volumen-Gasgemisch umsetzt.

3. Verfahren nach Anspruch 1, dadurch gekennzeichnet, dass man die Umsetzung bei Temperaturen von 10 bis 50 °C vornimmt.

4. Verfahren nach Anspruch 1, dadurch gekennzeichnet, dass die Verweilzeit 0,5 bis 5,0 Sekunden beträgt.

**Revendications**

1. Procédé pour la préparation de 1,2-dichloréthane par la mise en réaction d'éthylène avec le chlore en présence de catalyseurs, d'après lequel on introduit un mélange liquide finement divisé de 1,2-dichloréthane et de chlore à l'extrémité supérieure d'une zone de réaction dont la longueur est comprise entre 5 et 30 fois le diamètre de sa section et dans laquelle est contenu un mélange de gaz renfermant 0,3 à 30% en volume d'éthylène, 5 à 25 litres du mélange liquide correspondant à 1000 litres du mélange de gaz et le chlore étant présent dans le mélange liquide dans des quantités telles que 1 à 1,2 mol de chlore correspondent à 1 mol d'éthylène, et la température dans la zone de réaction étant maintenue entre 0 et 90 °C, caractérisé en ce que le diamètre des particules du mélange liquide est inférieur à 500 microns et en ce que le temps de séjour moyen du mélange de gaz est compris entre 0,2 et 10,0 secondes.

2. Procédé selon la revendication 1, caractérisé en ce qu'on fait réagir un mélange de gaz renfermant 0,5 à 10% en volume d'éthylène.

3. Procédé selon la revendication 1, caractérisé en ce qu'on mène la réaction à une température comprise entre 10 et 50 °C.

4. Procédé selon la revendication 1, caractérisé en ce que le temps de séjour se situe entre 0,5 et 5,0 secondes.

**Claims**

1. A process for the preparation of 1,2-dichloroethane by reacting ethylene with chlorine in the presence of a catalyst, in which a liquid mixture of 1,2-dichlorethane and chlorine is introduced at the upper end of a reaction zone whose length is from 5 to 30 times its diameter and which contains a gas mixture containing from 0,3 to 30 per cent by volume of ethylene, from 5 to 25 liters of the liquid mixture being employed per 1,000 liters of the gas mixture, and chlorine being present in the liquid mixture in such an amount that from 1 to 1.2 moles of chlorine are present per mole of ethylene, and the temperature in the reaction zone is kept at from 0 to 90 °C, wherein the diameter of the particles of the liquid mixture is less than 500 µ, and the average residence time of the gas mixture is from 0.2 to 10.0 seconds.

2. A process as claimed in claim 1, wherein a gas mixture containing from 0.5 to 10 per cent by volume of ethylene is reacted.

3. A process as claimed in claim 1, wherein the reaction is carried out at a temperature of from 10 to 50 °C.

4. A process as claimed in claim 1, wherein the residence time is from 0.5 to 5.0 seconds.